Europäisches Patentamt

European Patent Office

Office européen des brevets

(19)

(11) Veröffentlichungsnummer: **0 163 122 B1**

# EUROPÄISCHE PATENTSCHRIFT

(12)

(45) Veröffentlichungstag der Patentschrift: **04.03.92**

(21) Anmeldenummer: **85104967.6**

(22) Anmeldetag: **24.04.85**

(51) Int. Cl.⁵: **G01N 33/53**, G01N 33/74, G01N 33/78

(54) **Verfahren zur Bestimmung freier Substanzen in biologischen Flüssigkeiten.**

(30) Priorität: **27.04.84 DE 3415818**

(43) Veröffentlichungstag der Anmeldung:
**04.12.85 Patentblatt 85/49**

(45) Bekanntmachung des Hinweises auf die
Patenterteilung:
**04.03.92 Patentblatt 92/10**

(84) Benannte Vertragsstaaten:
**AT BE CH DE FR GB IT LI LU NL SE**

(56) Entgegenhaltungen:
**EP-A- 0 026 103
EP-A- 0 073 865
EP-A- 0 089 806
WO-A-85/00226
FR-A- 2 400 205**

(73) Patentinhaber: **Henning Berlin GmbH Chemie
und Pharmawerk
Komturstrasse 19-20
W-1000 Berlin 42(DE)**

(72) Erfinder: **Bienhaus, Gerhard
Drakestr. 21f
W-1000 Berlin 45(DE)**

(74) Vertreter: **Andrae, Steffen, Dr. et al
Patentanwälte Andrae, Flach, Haug, Kneissl
Balanstrasse 55
W-8000 München 90(DE)**

EP 0 163 122 B1

**Beschreibung**

Die vorliegende Erfindung betrifft ein Verfahren zur Bestimmung der freien Substanzen freies Trijodthyronin und freies Thyroxin in biologischen Flüssigkeiten, und zwar ein Verfahren, das üblicherweise als Immunoassay bezeichnet wird.

Derartige Verfahren existierten in vielfachen Abwandlungen und ihre Prinzipien sind dem Fachmann gut bekannt. Eine einfache Zusammenfassung der Grundlagen derartiger Verfahren findet sich z.B. in T.G. Cooper, Biochemische Arbeitsmethoden, S. 269 ff.

Grundsätzlich wird bei derartigen Verfahren so vorgegangen, daß man der biologischen Flüssigkeit, die die zu bestimmende Substanz enthält, zusetzt:

a) eine erste Komponente, die eine markierte Form der zu bestimmenden Substanz oder ein markiertes Derivat dieser Substanz enthält sowie

b) eine zweite Komponente, die spezifisch die zu bestimmende Substanz in unmarkierter Form sowie die erste Komponente gemäß a) bindet,

danach das erhaltene Gemisch für einen ausreichenden Zeitraum inkubiert, die bei der Umsetzung des Gemischs aus der zu bestimmenden Substanz und der ersten Komponente gemäß a) mit der zweiten Komponente gemäß b) gebildete gebundene Phase von dem restlichen Gemisch abtrennt, den Anteil der ersten Komponente in der gebundenen Phase anhand ihrer Markierung ermittelt und schließlich aus diesem ermittelten Anteil rechnerisch auf die Menge der zu bestimmenden freien Substanz in der biologischen Flüssigkeit rückschließt.

Üblicherweise sind die Substanzen, die nach derartigen Verfahren bestimmt werden, Antigene oder Haptene, insbesondere Hormone und ähnliche biologisch aktive Substanzen, und als zweite Komponente werden Antikörper gegen die jeweiligen Antigene oder Haptene verwendet.

Bei Zusatz der ersten Komponente, im folgenden kurz Tracer genannt, der im wesentlichen in gleicher Weise wie die zu bestimmende Substanz an die zweite Komponente, im folgenden Antikörper (Ak) genannt, bindet, kommt es zu einer Konkurrenz der zu bestimmenden Substanz und des Tracers um die Bindungsstellen des Antikörpers. Da sowohl der Tracer als auch der Antikörper in bekannter Konzentration zugesetzt werden, läßt sich aus dem Anteil des Tracers in der gebildeten gebundenen Phase Substanz/Tracer-Antikörper die Konzentration der zu bestimmenden Substanz in der biologischen Flüssigkeit ermitteln.

Der Tracer enthält Merkmale, die die Bestimmung seiner Konzentration mit physikalischen oder biochemischen Verfahren ermöglichen. In der bekanntesten Form als Radioimmunassay (RIA) enthält der Tracer ein radioaktives Atom. Das vorliegende Verfahren wird nachfolgend insbesondere in seiner Ausgestaltung als derartiger Radioimmunoassay beschrieben. Die Anwendung des erfindungsgemäßen Verfahrens ist jedoch nicht auf RIA's beschränkt, sondern es kann in wirksamer Weise auch mit enzymgekoppelten Tracern als Enzymimmunoassay, mit fluoreszierendem Tracer als Fluoreszenzimmunoassay oder mit einem lumineszierenden Tracer als Lumineszenzimmunoassay durchgeführt werden.

Von den den allgemeinen Stand der Technik bildenden Veröffentlichungen seien stellvertretend für zahlreiche andere Publikationen erwähnt: Ein Artikel von R.S. Yalow und S.A. Berson in: Nature, 184, S. 308 bis 312, die US-PS 36 54 090 und die DE-OSen 27 22 391, 32 05 506 und 29 21 781.

Während derartige Bestimmungsverfahren zur Bestimmung der Gesamtmenge der in einer biologischen Flüssigkeit vorliegenden Substanzen gut entwickelt und praktisch eingeführt sind, treten bei einer Reihe von physiologisch wichtigen Substanzen insofern Probleme auf, als diese Substanzen nebeneinander in einer freien Form und einer gebundenen Form an physiologische Bindeproteine gebunden vorliegen. Es hat sich nunmehr aufgrund medizinischer Befunde herausgestellt, daß physiologisch vorrangig der "freie " Anteil wichtig ist, so daß nach Verfahren zur Bestimmung ausschließlich dieses freien Anteils gesucht wurde. Es wurden bereits eine Reihe derartiger Verfahren entwickelt, die beispielsweise in einem Artikel von R. Ekins "The radioimmunoassay of free hormones in blood" in "Free Hormones in Blood", S. 73 bis 90, Elsevier Biomedical Press 1982 zusammengefaßt dargestellt werden. Derartige Verfahren sind auch in den europäischen Patentanmeldungen EP 0026103A1 und EP 0073865A1 näher beschrieben. Ein weiteres Verfahren, das aufgrund der speziellen Derivate der zu bestimmenden Substanz, die als Tracer verwendet werden, ebenfalls ein Verfahren zur Messung der freien Substanzen darstellt, ist in der DE-PS 28 18 644 beschrieben. In der Einleitung dieser Patentanmeldungen finden sich weitere Erläuterungen zur Bedeutung der Bestimmung freier Substanzen wie z.B. freier Hormone. Bei der Bestimmung derartiger "freier" Substanzen ergeben sich Probleme daraus, daß in der biologischen Flüssigkeit zwischen der gebundenen und freien Form einer Substanz ein Gleichgewicht herrscht, das durch den Zusatz des Tracers und das Binden der freien Substanz gestört wird. Der Tracer wird daher in sehr geringen Mengen zugesetzt. Durch Zusatz eines Tracers, der eine stärkere oder vergleichbare Bindungsneigung gegenüber den physiologischen Bindeproteinen aufweist, kann es jedoch dazu kommen, daß gebundene Substanz aus den Bindeproteinen freigesetzt wird und andererseits Tracer gebunden wird. Dabei kommt es zu einer Verminderung der zugesetzten Tracerkonzentration, ggf. begleitet von einer Erhöhung der Konzentration an freien Substanzen, und das bei dem Bestimmungsverfahren erhaltene Ergebnis ist verfälscht.

Zur Lösung diese Problems wurde in den bereits zitierten Anmeldungen versucht, Tracer zu entwickeln, die zwar gegenüber dem Antikörper die gleiche Bindungsneigung aufweisen wie die zu bestimmende Substanz, die jedoch gegenüber den Bindeproteinen sehr viel geringere Affinitäten zeigen. Bei dem in der genannten EP 0026103 beschriebenen Verfahren werden zur Bestimmung der Schilddrüsenhormone Thyroxin (T4) oder Trijodthyronin (T3) als Tracer derivatisierte Formen dieser Schilddrüsenhormone verwendet, die sich durch chemische Modifizierungen an der Carboxylund/oder Aminogruppe von T4 bzw. T3 unterscheiden. Ferner kann an Stelle der das Hormon bildenden L-Form auch die D-Form verwendet werden. Bei dem in der EP 0073865 beschriebenen Verfahren werden Tracer verwendet, die Schilddrüsenhormonderivate bzw. -analoga enthalten, die kovalent gebundene Polyamine, insbesondere als Komplexbildner bekannte Substanzen wie EDTA, DTPA und NTA enthalten. Die in der DE-PS 28 18 644 beschriebenen Derivate, die in der 3'-Stellung ein Bromatom aufweisen, umfassen ebenfalls entsprechende Derivate, die bei ihrer Verwendung als Tracer nicht oder kaum an die Bindeproteine binden, dagegen gegenüber der zweiten Komponente (Ak) eine Bindefähigkeit aufweisen, die der der zu bestimmenden Substanz vergleichbar ist. Bei ihrer Verwendung werden somit ebenfalls die freien Formen bestimmt. Soweit in dieser Anmeldung im Zusammenhang mit der ersten Komponente davon gesprochen wird, daß diese markierte Derivate der zu bestimmenden Substanz enthält, sind damit insbesondere Derivate gemeint, wie sie in der DE-PS 28 18 644 beschrieben sind, sowie ferner in der EP 0026103 und der EP 73865. Durch die Derivatisierung bzw. die Verwendung der D-Form konnte erreicht werden, daß derartige Tracer bei weitgehender Erhaltung der Bindungsfähigkeit an die Antikörper eine geringere Bindungsneigung gegenüber den Serumsproteinen, die als Thyroxin-bindendes Globulin (TBG) und Thyroxin-bindendes Prealbumin (TBPA) bezeichnet werden, aufweisen. Es wurde angenommen, daß auch das dritte bekannte physiologische Bindeprotein, das Albumin, nicht zu einer Verfälschung der Meßergebnisse führt. Es hat sich nunmehr aber herausgestellt, daß auch bei einem derartigen Zweikomponentensystem Fehler bei der Bestimmung der freien Schilddrüsenhormone dadurch auftreten können, daß die Überschüsse an physiologischen Bindeproteinen dazu führen, daß der Tracer im Sinne einer "Rückbindung" an diese Bindeproteine gebunden wird, wodurch seine Konzentration herabgesetzt wird und die Meßergebnisse verfälscht werden. Diese "Tracer-Rückbindung" führte vor allem bei abnormalen Bindungsproteinverteilungen in einigen Proben zu falschen Ergebnissen. Es kann in diesem Zusammenhang auf verschiedene wissenschaftliche Artikel verwiesen werden:

J.R. Stockigt, V. Stevens, E.L. White und J.W. Barlow in Clin. Chem. 29/7, 1408 bis 1410 (1983); N. Amino et al in Clin. Chem. 29/2, (1983), S. 321 bis 325. In diesen Arbeiten konnte gezeigt werden, daß insbesondere der Albuminanteil in der biologischen Flüssigkeit die Bindung des Tracers an den Antikörper behindert. Ferner ist es als genereller Mangel bei einer Verwendung von derivatisierten Formen als Tracer anzusehen, daß jede Derivatisierung in der Regel eine von 100% abweichende Kreuzreaktivität zur Folge hat. Ein Verfahren, das eine Bestimmung der freien Form unter Verwendung eines Tracers, der bis auf die Markierung mit der zu bestimmenden Substanz identisch ist, ermöglicht, weist somit einen Vorteil auf, der den bisher bekannten Verfahren fehlt.

Es ist Aufgabe der vorliegenden Erfindung, ein neues Bestimmungsprinzip zur Bestimmung der freien Hormone Thyroxin und Trijodthyronin anzugeben, bei dem es zu keiner Verfälschung der Meßergebnisse durch Tracer-Rückbindung kommt und das es außerdem gestattet, die bei der Verwendung von derivatisierten Tracern durch Rückbindung auftretenden Fehler zu vermeiden.

Diese Aufgabe wird erfindungsgemäß gelöst durch ein Verfahren zur Bestimmung von freiem Trijodthyronin oder freiem Thyroxin in biologischen Flüssigkeiten in Gegenwart von einem oder mehreren physiologischen Bindeproteinen für die genannten freien Substanzen, bei dem der biologischen Flüssigkeit zugesetzt werden:

a) eine erste Komponente, die eine markierte Form der zu bestimmenden Substanz oder ein markiertes Derivat dieser Substanz enthält sowie
b) eine zweite Komponente, die spezifisch die zu bestimmende Substanz in unmarkierter Form sowie die erste Komponente gemäß a) bindet,

wonach das erhaltene Gemisch für einen ausreichenden Zeitraum inkubiert wird, die bei der Umsetzung des Gemischs aus der zu bestimmenden Substanz und der ersten Komponente gemäß a) mit der zweiten Komponente gemäß b) gebildete gebundene Phase von dem restlichen Gemisch abgetrennt wird, der Anteil der ersten Komponente in der gebundenen Phase anhand ihrer Markierung ermittelt wird und schließlich aus diesem ermittelten Anteil rechnerisch die Menge der zu bestimmenden freien Substanz in der biologischen Flüssigkeit ermittelt wird, dadurch gekennzeichnet, daß der biologischen Flüssigkeit zusätzlich c) eine dritte Komponente zugesetzt wird, die aus Schilddrüsenhormonanaloga und/oder wäßrigen Salzlösungen einer Konzentration entsprechend von 0,25 bis 1,8 M ausgewählt ist.

Zur Bedeutung der Angaben in den Patentansprüchen wird auf die vorliegende Beschreibung verwiesen, der weitere Einzelheiten für die Durchführung des erfindungsgemäßen Verfahrens entnommen werden können.

Das erfindungsgemäße Verfahren unterscheidet sich von den herkömmlichen Verfahren, bei denen nur 2 Komponenten verwendet werden, nämlich der "Tracer" und der "Antikörper", dadurch, daß eine dritte Komponente zugesetzt wird, die spezifisch eine Rückbindung des Tracers an die Bindeproteine verhindert. Dadurch kommt es zu keiner Veränderung der Tracer-Konzentration durch Rückbindung, und die erhaltenen Ergebnisse spiegeln die tatsächlich vorliegende Konzentration der freien Hormone besser wieder. Grundsätzlich unterscheidet sich das erfindungsgemäße Verfahren von den vorbekannten Verfahren dadurch, daß nicht der Tracer oder der Antikörper verändert werden, sondern daß gezielt auf die Bindeproteine, insbesondere auf die im Überschuß in freier Form in der biologischen Flüssigkeit vorliegenden Bindeproteine, eingewirkt wird, so daß diese die Messung nicht verfälschen. Die durch die dritte Komponente erzielte Veränderung der Bindeproteine läßt sich allgemein als Herabsetzung oder Eliminierung ihrer Bindungsaffinität gegenüber dem Tracer beschreiben. Diese Verminderung der Bindungsaffinität kann prinzipiell auf verschiedene Weise erfolgen, wobei einmal eine "Neutralisierung" der Bindeproteine durch die dritte Komponente erfolgt, die nicht zum eigentlichen Meßsystem gehört und an die freien Bindeproteine bindet, so daß eine Tracer-Rückbindung verhindert wird. Es ist jedoch genauso möglich, die Bindungsaffinität der Bindeproteine an sich dadurch herabzusetzen, daß bei Bedingungen gearbeitet wird, die für die Ausbildung einer Bindung Bindeprotein/Tracer ungünstig sind.

Die dritte Komponente kann der zu bestimmenden Probe vor der Zugabe des Tracers zugesetzt werden, jedoch auch mit diesem zusammen. Die Möglichkeit, direkt das zu bestimmende Hormon in der markierten Form einzusetzen, stellt sicher, daß Kreuzreaktivitäten von 100% erreicht werden können.

Durch die Verhinderung der Tracer-Rückbindung durch Zusatz einer dritten Komponente konnte überraschenderweise eine entscheidende Verbesserung der Bestimmungsgenauigkeit der freien Hormone fT3 und ft4 erzielt werden. Diese Ergebnisse waren insofern unerwartet, als zu befürchten war, daß bei dem Versuch einer Beeinflussung der Bindungsaffinität der Bindeproteine eine Freisetzung der gebundenen Form der zu bestimmenden Substanz erfolgt. Die vorliegenden praktischen Ergebnisse haben gezeigt, daß diese Befürchtung nicht gerechtfertigt ist, wenn Art und Konzentration der dritten Komponente wie in Anspruch 1 angegeben ausgewählt werden.

Das erfindungsgemäße Verfahren kann so durchgeführt werden, daß einmal als Tracer direkt die markierte Form der zu bestimmenden Substanz verwendet wird, und daß die dritte Komponente durch Verminderung der Tracer-Rückbindung das Gleichgewicht freie zu bestimmende Substanz/an das Bindeprotein gebundene Substanz/ Bindeprotein mit freien Bindungsstellen so beeinflußt, daß bei dem Immunoassay nur die freie Form gemessen wird.

Das erfindungsgemäße Verfahren kann ferner auch als Verbesserung des Immunoassays mit dem derivatisierten Tracer, z.B. gemäß DE-PS 28 18 644, EP 0026103 oder EP 00 73865 verwendet werden, indem es in einem solchen System die Rückbindung verhindert.

Es hat sich gezeigt, daß das erfindungsgemäße Verfahren eine Bestimmung des freien T3 ermöglicht, ohne daß ein derivatisierter Tracer verwendet werden muß. Der Tracer (die erste Komponente gemäß a)) kann somit ein markiertes T3 sein, insbesondere ein T3, das ein radioaktives Jodisotop enthält, beispielsweise 131J und insbesondere 125J. 125J-markiertes T3 ist als Tracer bevorzugt. Eine besonders bevorzugte Ausführungsform betrifft ferner die Verwendung eines Lumineszenztracers, insbesondere gemäß DE-OS 29 21 781.

Ein erfindungsgemäßer Immunoassay zur Bestimmung des freien T3 enthält somit außer den üblichen Bestandteilen 125J-markiertes T3 als erste Komponente und einem T3-Antiserum (T3-Antikörper) als zweite Komponente eine dritte Komponente, die vorzugsweise eines der Schilddrüsenhormonanaloga 3,3',5-Trijodthyronin (rT3), 3',5'-Dijodthyronin, 3,5-Dijod-3'-isopropyl-thyronin, L-Thyroxin, 3,3',5,5'-Tetrajodthyreoessigsäure, 3',5'-Dijodthyreoessigsäure oder 3,3',5'-Trijodthyreoessigsäure ist. Soweit die genannten Verbindungen in L und D-Form vorliegen können, können außer den L-Formen auch die D-Formen verwendet werden. Die Schilddrüsenhormonanaloga werden in solchen Konzentrationen eingesetzt, daß einerseits die Rückbindung des markierten T3-Tracers verhindert wird, andererseits kein gebundenes T3 freigesetzt wird. Als geeignete Mengen haben sich, bezogen auf jeweils 0,1ml Humanserumeinsatz, 0,05 bis 1,5 nmol, bevorzugt 0,1 bis 1 nmol des Hormonanalogen erwiesen. Praktisch sehr befriedigende Ergebnisse werden dabei in der Regel bei ca. 0,8 nmol erhalten.

Es konnte bei einem derartigen speziellen Assaysystem mittels Charcoaltrennung gezeigt werden, daß in einem derartigen Dreikomponentensystem eine Tracer-Rückbindung unterdrückt ist.

Klinische Tests ergaben, daß die Radioimmunoassays auf Basis der erfindungsgemäßen Dreikomponentensysteme Ergebnisse liefern, die herkömmlichen Radioimmunoassays vergleichbar sind, wobei in speziellen Fällen Fehler der herkömmlichen Systeme vermieden werden.

Ein weiteres bevorzugtes Verfahren betrifft die Bestimmung von freiem Thyroxin (FT4) aus Humanserum. Bei einem solchen System wurde gezeigt, daß die bekannte Derivatisierung des Thyroxin-Tracers zur Verhinderung der Rückbindung an das Serumsprotein TBG falsche Werte lieferte, da eine Tracer-Rückbindung an eine weitere bindende Komponente erfolgte, nämlich an das Albumin.

Diese Fehlerquelle konnte überraschenderweise bei dem erfindungsgemäßen Verfahren eliminiert werden, indem als dritte Komponente eine wäßrige Salzlösung hoher Ionenstärke verwendet wurde. Geeignete Salze sind beispielsweise Kalium-, Natrium- und Magnesiumchlorid, Kalium-, Natriumbromid, Kalium-, Natriumphosphat, Kalium-, Natriumsulfat und ähnliche ionisierbare Salze, die in hohen Konzentrationen, insbesondere von 0,25 bis 1,8 M, bevorzugt 0,4 bis 1,5 M, beispielsweise in der Größenordnung von 1M, verwendet werden. Die Anwesenheit derartiger Salz- oder Pufferlösungen mit hoher Ionenstärke verhindert offensichtlich ebenfalls eine Rückbindung an die physiologischen Serumsproteine.

Bei einem erfindungsgemäßen Verfahren zur Bestimmung von freiem T4 wird somit außer den bekannten Komponenten 125J-markiertes Thyroxin-Derivat (insbesondere ein Derivat gemäß DE-PS 28 18 644) und T4-Ankikörper eine dritte Komponente verwendet, die eine Salzlösung hoher Ionenstärke ist. Als dritte Komponente kann auch, ggf. zusätzlich, eines der weiter oben näher bezeichneten Schilddrüsenhormonanaloga angewandt werden. Orientierende Versuche zeigten, daß es auch im Falle der Bestimmung von freiem T4 möglich ist, als Tracer ein radioaktiv markietes, nichtderivatisiertes T4 zu verwenden.

Bei den erfindungsgemäßen Verfahren erfolgt die Trennung der gebundenen Phase von der restlichen freien Fraktion nach den üblichen klassischen Verfahren, beispielsweise durch Solidphase-Techniken (Mikropartikeln, Makrokugeln oder beschichteten Röhrchen) oder als präpräzipitiertes Antiserum.

Nachfolgend wird die vorliegende Erfindung anhand von konkreten Anwendungsbeispielen unter Bezugnahme auf zwei Figuren noch näher erläutert.

Es zeigen:

Fig.1 eine typische Standardkurve für einen FT3-Test

Fig.2 eine typische Standardkurve für einen FT4-Test

Beispiel 1

3-Komponentensystem zur Bestimmung von freiem Trijodthyronin:

Herstellung der Assay-Komponenten:

1) Standards

Standards in Humanserum werden nach Standardverfahren hergestellt und mit einer Primärmethode wie Gleichgewichtsdialyse nach Ellis S. und Ekins R. (1973, Acta Endocrinologica (Kbh) Suppl. 177; 106) kalibriert. Die Konzentrationen für den freien Anteil an Trijodthyronin wurden für dieses Beispiel wie folgt bestimmt:

Nullserum: Weitgehend frei von Trijodthyronin (ca. 99%).
Standard 1: 1,1 pmol T3/l
Standard 2: 2,2 pmol T3/l
Standard 3: 4,4 pmol T3/l
Standard 4: 8,8 pmol T3/l
Standard 5: 17,6 pmol T3/l
Standard 6: 35,2 pmol T3/l

Die Standards müssen so gewählt werden, daß sie sich über den physiologisch relevanten Messbereich erstrecken.

2) Tracer

Ein 125J-markiertes Trijodthyronin als Tracer (spezifische Aktivität ca. 2300 Ci/g) in Natriumbarbitalpuffer (50 mmol/l; pH 8,4) wird analog zu H. Rokos und H. Steinmaus (1981, J. Clin. Chem. Clin. Biochem. Vol. 19, pp. 191-194) hergestellt.

3) Dritte Komponente

Eine Lösung von 3 microgr/ml L-3',5' Dijodthyronin (Fa. Henning Berlin GmbH) in Natriumbarbitalpuffer (50 mmol/l; pH 8,4) wurde als dritte Komponente gemäß c) nach Anspruch 2 verwendet.

4) Antiserum

Zur Vereinfachung der Handhabung des Tests wird ein T3-Antiserum als Festphasen-gekoppeltes Antiserum vom Kaninchen verwendet: Als Konjugat zur Immunisierung des Versuchstieres wurde ein Trijodthyronin-Rinderserumkonjugat benutzt. Das daraus gewonnene Antiserum zeigte eine Bindecharakteristik von ca. 65% Trijodthyronintracer bei einer Verdünnung von 1:30.000. Kreuzreaktivitäten zu anderen Schilddrüsenhormonanaloge lagen unter 0,18%.

Dieses Antiserum wird nach Gooch G.T. et al. (1983 Applied Biochemistry and Biotechnology Vol. 8 105-114) an Micropartikel aus Polyamid kovalent gebunden und als Suspension in Kaliumphosphatpuffer (50 mmol/l; pH 7,4) mit 2% Ovalbumin in das Testsystem eingesetzt.

Testdurchführung:

Zur Durchführung des Testes werden in verschiedene Teströhrchen jeweils 0,1 ml Standards bzw. unbekannte Serumproben pipettiert. Danach wird nacheinander je 0,1 ml Komponente III (wie oben), 0,1 ml T3-Tracer (ca. 30.000 Impulse pro Minute entspr. ca. 750 Bq) und 1 ml FT3-Antiserum-Suspension (wie oben) in jedes Röhrchen zugesetzt und 2 Std. bei 37°C in einem Brutschrank oder Wasserbad inkubiert. Anschließend werden die Röhrchen mit mindestens 1000 g für 5 Min. zentrifugiert und die im Überstand befindliche freie Phase abgesaugt. Die im Pellet gebundene Menge an Tracer wird in einem Gammazähler gemessen und entspricht der gebundenen Phase.

Testergebnisse:

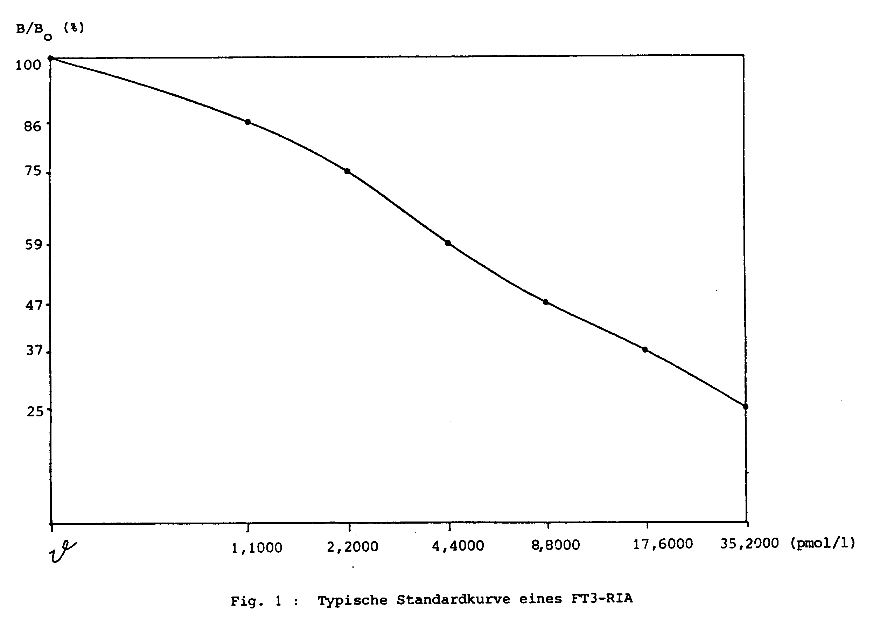

Die Messwerte der Standards ergeben in einer für den Radioimmunoassay typischen Auswertung $(B/B_O)$ und halblogarithmischem Auftragung eine Standardkurve (Abb. 1), an der die Konzentrationen der unbekannten Proben abgelesen werden können.

Klinische Wertigkeit:

Zur Überprüfung der klinischen Wertigkeit der FT3-Bestimmung wurden drei Patientenkollektive herangezogen, deren Schilddrüsenstatus an Hand von klinischen Anzeichen, sowie den derzeit üblichen Laborparametern wie Gesamt-thyroxin, Gesamt-Trijodthyronin sowie dem TSH/TRH-Test (P. Pfannenstiel, "Diagnostik von Schilddrüsenerkrankungen", 4. Auflage, ISBN 3-87018-031-5) eindeutig als "hyperthyreot", "euthyreot" und "hypothyreot" einzustufen waren.

Die Mittelwerte aller FT3-Bestimmungen und die entsprechenden 2s-Abweichungen liegen bei:

1,15 ± 1,6 pmol/l für hypothyreote Personen (n = 20)

5,42 ± 2,4 pmol/l für euthyreote Personen (n = 42)

15,8 ± 10 pmol/l für hyperthyreote Personen (n = 18)

Damit ist eine hinreichende Klassifizierung des Schilddrüsenstatus durch die erfindungsgemäße FT3-Bestimmung mit folgenden Normgrenzen gegeben:

Hypothyreosen: < 3,2 pmol/l
Euthyreosen: 3,2-9 pmol/l
Hyperthyreosen: > 9 pmol/l

In einigen speziellen Serumproben mit veränderten Schilddrüsenhormon-Bindeproteinen wie sie z.B. auch bei schwangeren Frauen auftreten können, zeigte das erfindungsgemäße Verfahren bessere Korrelationen der FT3-Werte zu dem diagnostizierten Schilddrüsenstatus als herkömmliche Zweikomponentenverfahren.

Beispiel 2

3-Komponentensystem zur Bestimmung von freiem Thyroxin:

Herstellung der Assay-Komponenten:

1) Standards

Standards in Humanserum werden nach Standardverfahren hergestellt und mit einer Primärmethode wie Gleichgewichtsdialyse nach S. Ellis und R. Ekins (1973, Acta Endocrinologica (Kbh) Suppl. 177; 106) kalibriert. Die Konzentrationen für den freien Anteil von Thyroxin wurden für dieses Beispiel wie folgt bestimmt:

Nullserum: Weitgehend frei von Thyroxin (ca. 99%).

Standard 1: 2 pg T4/ml

Standard 2: 4 pg T4/ml

Standard 3: 8 pg T4/ml

Standard 4: 16 pg T4/ml

Standard 5: 32 pg T4/ml

Standard 6: 64 pg T4/ml

Die Standards müssen so gewählt werden, daß sie sich über den physiologisch relevanten Messbereich erstrecken.

2) Tracer

Ein 125J-markiertes Thyroxinderivat, gemäß DEO 28 18 644 N-Acetyl-3'-brom-5'-(jod-125)-3,5-dijod-L-thyronin, (spezifische Aktivität ca. 2300 Ci/g) in Natriumphosphatpuffer (50 mmol/l; pH 7,4) mit 1 mol/l Kaliumchlorid wird, als Tracer in diesem Beispiel benutzt.

Zu vergleichbaren Ergebnissen kommt man auch mit anderen N-substituierten Niederalkanoyl-Thyroxinderivate, sowie mit Thyroxinderivaten wie in EP 0026103 und EP 0073865 beschrieben.

3) Dritte Komponente

Als dritte Komponente nach Anspruch 2 dient in diesem Beispiel die Salzlösung (1mol/l Kaliumchlorid als Zusatz in der Tracerlösung 2), die mit dem Tracer zugleich zugegeben werden kann.

4) Antiserum

Zur Vereinfachung der Handhabung des Tests wird ein T4-Antiserum als Festphasen-gekoppeltes Antiserum vom Kaninchen verwendet: Als Konjugat zur Immunisierung des Versuchstieres wurde ein Thyroxin-Rinderserumkonjugat benutzt. Das daraus gewonnene Antiserum zeigte eine Bindecharakteristik von ca. 65% Thyroxintracer bei einer Verdünnung von 1:800. Kreuzreaktivitäten zu anderen Schilddrüsenanaloga lagen unter 0,1%, die Kreuzreaktivität zu dem in diesem Beispiel als Tracer verwendeten Thyroxinderivat lag bei 102%.

Dieses Antiserum wird nach G.T. Gooch et al. (1983 Applied Biochemistry and Biotechnology Vol.8 105-114) an Micropartikel aus Polyamid kovalent gebunden und als Suspension in Kaliumphosphatpuffer (50 mmol/l; pH 7,4) mit 2% Ovalbumin in das Testsystem eingesetzt.

Testdurchführung:

Zur Durchführung des Testes werden in verschiedenen Teströhrchen jeweils 0,1 ml Standards bzw. unbekannte Serumproben pipettiert.

Danach wird 0,1 ml Tracer (ca. 30 000 Impulse pro Minute entspr. ca. 750 Bq) und 1 ml FT4-Antiserum-Suspension (wie oben) in jedes Röhrchen zugesetzt und eine Stunde bei Raumtemperatur inkubiert. Anschließend werden die Röhrchen mit mindestens 1000 g für 5 Min. zentrifugiert und die im Überstand befindliche freie Phase abgesaugt. Die im Pellet gebundene Menge an Tracer wird in einem Gammazähler gemessen und entspricht der gebundenen Phase.

Testergebnisse:

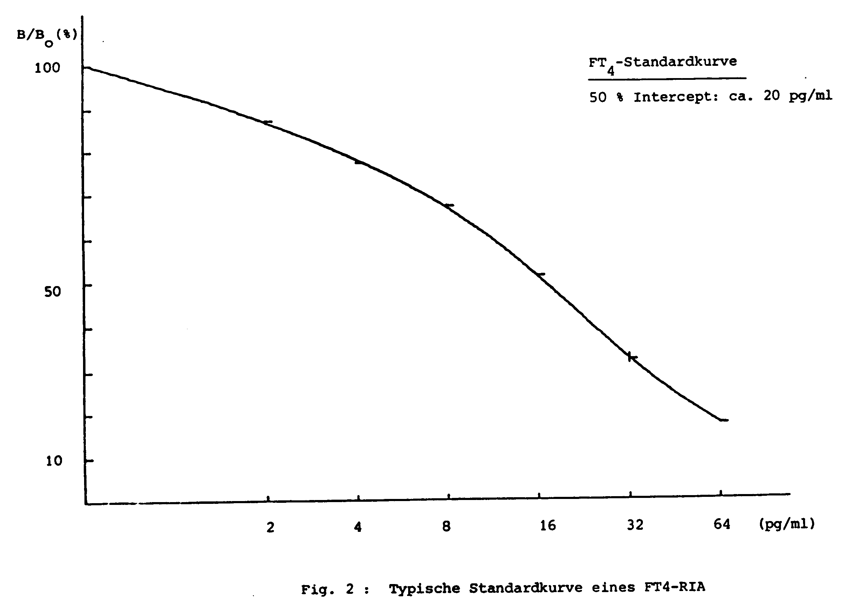

Die Messwerte der Standards ergeben in einer für den Radioimmunoassay typischen Auswertung ($B/B_O$) und halblogarithmischen Auftragung eine Standardkurve (Abb. 2), an der die Konzentrationen der unbekannten Proben abgelesen werden können.

Klinische Wertigkeit:

Zur Überprüfung der klinischen Wertigkeit der FT4-Bestimmung wurden drei Patientenkollektive herangezogen, deren Schilddrüsenstatus an Hand von klinischen Anzeichen, sowie den derzeit üblichen Laborparametern wie Gesamtthyroxin, Gesamt-Trijodthyronin sowie dem TSH/TRH-Test (P. Pfannenstiel, "Diagnostik von Schilddrüsenerkrankungen", 4. Auflage, ISBN 3-87018-031-5) eindeutig als "hyperthyreot", "euthyreot" und "hypothyreot" einzustufen waren.

Die Mittelwerte aller FTA-Bestimmungen und die entsprechenden 2s-Abweichungen liegen bei:
6 ± 2,5 pg/ml für hypothyreote Personen (n = 23)
14 ± 3 pg/ml für euthyreote Personen (n = 143)
26 ± 8 pg/ml für hyperthyreote Personen (n = 43)

Damit ist eine hinreichende Klassifizierung des Schilddrüsenstatus durch die erfindungsgemäße FT4-Bestimmung mit folgenden Normgrenzen gegeben:
Hypothyreosen: < 9 pg/ml
Euthyreosen: 10-19 pg/ml
Hyperthyreosen: > 20 pg/ml

**Patentansprüche**

1. Verfahren zur Bestimmung von freiem Trijodthyronin oder freiem Thyroxin in biologischen Flüssigkeiten in Gegenwart von einem oder mehreren physiologischen Bindeproteinen für die genannten freien Substanzen, bei dem der biologischen Flüssigkeit zugesetzt werden:

a) eine erste Komponente, die eine markierte Form der zu bestimmenden Substanz oder ein markiertes Derivat dieser Substanz enthält sowie

b) eine zweite Komponente, die spezifisch die zu bestimmende Substanz in unmarkierter Form sowie die erste Komponente gemäß a) bindet,

wonach das erhaltene Gemisch für einen ausreichenden Zeitraum inkubiert wird, die bei der Umsetzung des Gemischs aus der zu bestimmenden Substanz und der ersten Komponente gemäß a) mit der zweiten Komponente gemäß b) gebildete gebundene Phase von dem restlichen Gemisch abgetrennt wird, der Anteil der ersten Komponente in der gebundenen Phase anhand ihrer Markierung ermittelt wird und schließlich aus diesem ermittelten Anteil rechnerisch die Menge der zu bestimmenden freien Substanz in der biologischen Flüssigkeit ermittelt wird, **dadurch gekennzeichnet,** daß der biologischen Flüssigkeit zusätzlich c) eine dritte Komponente zugesetzt wird, die aus Schilddrüsenhormonanaloga und/oder wäßrigen Salzlösungen einer Konzentration entsprechend von 0,25 bis 1,8 M ausgewählt ist.

2. Verfahren nach Anspruch 1, **dadurch gekennzeichnet,** daß die Schilddrüsenhormonanaloga L-3,3',5'-Trijodthyronin, L-3',5'-Dijodthyronin, L-3,5-Dijod-3'-isopropylthyronin, 3,3',5,5'-Tetrajodthyreoessigsäure, 3',5'-Dijodthyreoessigsäure, 3,3',5'-Trijodthyreoessigsäure oder, im Falle einer Bestimmung von freiem Trijodthyronin, L-Thyroxin sind und in Mengen von 0,05 bis 1,5 nmol, bezogen auf 0,1 ml Humanserum als biologische Flüssigkeit verwendet werden.

3. Verfahren nach Anspruch 1, **dadurch gekennzeichnet**, daß als wäßrige Salzlösungen Lösungen von Kaliumchlorid, Natriumchlorid und/oder Magnesiumchlorid oder entsprechend hochkonzentrierte Pufferlösungen verwendet werden.

4. Verfahren nach einem der Ansprüche 1 bis 3 zur Bestimmung von freiem Trijodthyronin, **dadurch gekennzeichnet**, daß die erste Kom-ponente ein radioaktiv markiertes Trijodthyro-nin ist, die zweite Komponente ein Trijodthyronin-Antiserum ist und die dritte Komponente ein Schilddrüsenhormonanalgon ist.

5. Verfahren nach einem der Ansprüche 1 bis 3 zur Bestimmung von freiem Trijodthyronin, **dadurch gekennzeichnet,** daß die erste Kom-ponente ein radioaktiv markiertes Trijodthyron-inderivat mit verminderter Bindungsfähigkeit an wenigstens eines der physiologischen Bindeproteine ist, daß die zweite Komponente ein Trijodthyronin-Antiserum ist und die dritte Komponente ein Schilddrüsenhormonanalogon ist.

6. Verfahren nach Anspruch 4 oder Anspruch 5, **dadurch gekennzeichnet,** daß die erste Komponente ein mit $^{125}$J markiertes Trijodth-yronin oder Trijodthyroninderivat ist.

7. Verfahren nach einem der Ansprüche 1 bis 3 zur Bestimmung von freiem Thyroxin, **dadurch gekennzeichnet**, daß die erste Komponente ein radioaktiv markiertes Thyroxin ist, die zweite Komponente ein Thyroxin-Antiserum ist und die dritte Komponente ein Schilddrüsenhormonanalogon und/oder eine Salzlösung ist.

8. Verfahren nach einem der Ansprüche 1 bis 3 zur Bestimmung von freiem Thyroxin, **dadurch gekennzeichnet,** daß die erste Komponente ein markiertes Thyroxinderivat mit ver-minderter Bindefähigkeit gegenüber mindes-tens einem der physiologischen Bindeprotei-ne ist, die zweite Komponente ein Thyroxin-Antiserum ist und die dritte Komponente ein Schilddrüsenhormonanalogon und/oder eine Salzlösung ist.

9. Verfahren nach einem der Ansprüche 7 oder 8, **dadurch gekennzeichnet,** daß die erste Komponente mit $^{125}$J radioaktiv markiert ist.

10. Verfahren nach einem der Ansprüche 1 bis 3 oder 8, **dadurch gekennzeichnet,** daß die erste Komponente mit einer an sich bekannten, zur Lumineszenz befähigten Gruppe markiert ist.

## Claims

1. Process for the determination of free triiodothyronine or free thyroxine in biological fluids in the presence of one or more physiological binding proteins for the named free substances, wherein there is added to the biological fluid:

   a) a first component which contains a marked form of the substance to be determined or a marked derivative of this substance, and

   b) a second component which specifically combines with the substance to be determined in the unmarked form as well as with the first component according to a), and thereafter incubating the mixture so obtained for a sufficient period, followed by the separation from the remainder of the mixture of the bound phase formed by reaction of that mixture containing the substance which is to be determined and the first component according to a) with the second component according to b), determining the amount of the first component in the bound phase by means of its marking and then finally using this amount to calculate the amount of the free substance which is to be determined in the biological fluid, characterised in that, a third component c) chosen from analogues of thyroid gland hormones and/or aqueous salt solutions with a concentration corresponding to 0.25 to 1.8 M is additionally added to the biological fluid.

2. Process according to Claim 1, characterised in that, the analogues of thyroid gland hormones L-3,3',5'-triiodothyronine, L-3',5'-diiodothyronine, L-3,5-diiodo-3'-isopropylthyronine, 3,3',5,5'-tetraiodo-thyreo-acetic acid, 3',5'-diiodothyreo-acetic acid, 3,3',5'-triiodo-thyreo-acetic acid, or L-thyroxine in the case of the determination of free triiodothyronine, are used in amounts from 0.05 to 1.5 nmol based on 0.1 ml of human serum as the biological fluid.

3. Process according to Claim 1, characterised in that, solutions of potassium chloride, sodium chloride and/or magnesium chloride or suitable buffer solutions of high concentration are used as the aqueous salt solutions.

4. Process according to one of the Claims 1 to 3 for the determination of free triiodothyronine, characterised in that, the first component is a radioactively marked triiodothyronine, the second component is a triiodothyronine antiserum and the third component is an analogue of a thyroid gland hormone.

5. Process according to one of the Claims 1 to 3 for the determination of free triiodothyronine, characterised in that, the first component is a radioactively marked triiodothyronine derivative having a reduced bonding capability towards at least one of the physiological binding proteins, the second component is a triiodothyronine antiserum and the third component is an analogue of a thyroid gland hormone.

6. Process according to Claim 4 or Claim 5, characterised in that, the first component is a $^{125}$I-marked triiodothyronine or triiodothyronine derivative.

7. Process according to one of the Claims 1 to 3 for the determinaton of free thyroxine, characterised in that, the first component is a radioactively marked thyroxine, the second component is a thyroxine antiserum and the third component is an analogue of a thyroid gland hormone and/or a salt solution.

8. Process according to one of the Claims 1 to 3 for the determination of free thyroxine, characterised in that, the first component is a marked thyroxine derivative having a reduced bonding capability towards at least one of the physiological binding proteins, the second component is a thyroxine antiserum and the third component is an analogue of a thyroid gland hormone and/or a salt solution.

9. Process according to one of the Claims 7 or 8, characterised in that, the first component is radioactively marked with [125]I.

10. Process according to one of the Claims 1 to 3 or 8, characterised in that, the first component is marked with a group known per se which is capable of luminescence.

**Revendications**

1. Méthode de dosage de triiodothyronine libre ou de thyroxine libre dans des liquides biologiques en présence d'une ou plusieurs protéines physiologiques de liaison pour les substances libres mentionnées, dans laquelle le liquide biologique est additionné :

    a) d'un premier composant qui contient une forme marquée de la substance à doser ou un dérivé marqué de cette substance ainsi que,

    b) d'un second composant qui se lie de façon spécifique à la substance à doser sous une forme non marquée ainsi qu'au premier composant selon a),

après quoi le mélange obtenu est mis en incubation pendant un intervalle de temps suffisant, la phase liée formée lors de la réaction du mélange de la substance à doser et du premier composant selon a) avec le second composant selon b) est séparée du mélange restant, la fraction du premier composant dans la phase liée est dosée à l'aide de son marquage et finalement la quantité de substance libre à doser dans le liquide biologique est calculée d'après cette fraction déterminée, caractérisée en ce qu'on ajoute en supplément au liquide biologique c) un troisième composant qui est choisi entre des analogues d'hormones thyroïdiennes et/ou des solutions aqueuses de sels dont la concentration correspond à 0,25-1,8 M.

2. Méthode suivant la revendication 1, caractérisée en ce que les analogues d'hormones thyroïdiennes sont la L-3,3',5'-triiodothyronine, la L-3',5'-diiodothyronine, la L-3,5-diiodo-3'-isopropylthyronine, l'acide 3,3',5,5'-tétraiodothyréo-acétique, l'acide 3',5'-diiodothyréo-acétique, l'acide 3,3',5'-triiodothyréo-acétique ou, dans le cas d'un dosage de triiodothyronine libre, la L-thyroxine, et sont utilisés en quantités de 0,05 à 1,5 nmole par rapport à 0,1 ml de sérum humain comme liquide biologique.

3. Méthode suivant la revendication 1, caractérisée en ce qu'on utilise comme solutions aqueuses de sels des solutions de chlorure de potassium, de chlorure de sodium et/ou de chlorure de magnésium ou des solutions tampons à forte concentration correspondante.

4. Méthode suivant l'une des revendications 1 à 3 pour le dosage de triiodothyronine libre, caractérisée en ce que le premier composant est une triiodothyronine marquée par la radio-activité, le deuxième composant est un antisérum de triiodothyronine et le troisième composant est un analogue d'hormones thyroïdiennes.

5. Méthode suivant l'une des revendications 1 à 3 pour le dosage de triodothyronine libre, caractérisée en ce que le premier composant est un dérivé de triiodothyronine marqué par la radio-activité de capacité de liaison réduite envers au moins l'une des protéines de liaison physiologiques, le deuxième composant est l'antisérum de triiodothyronine et le troisième composant est un analogue d'hormones thyroïdiennes.

6. Méthode suivant la revendication 4 ou la revendication 5, caractérisée en ce que le premier composant est une triiodothyronine ou un dérivé de triiodothyronine marqué au $^{125}$I.

7. Méthode suivant l'une des revendications 1 à 3 pour le dosage de thyroxine libre, caractérisée en ce que le premier composant est une thyroxine marquée par la radio-activité, le deuxième composant est un antisérum de thyroxine et le troisième composant est un analogue d'hormones thyroïdiennes et/ou une solution de sel.

8. Méthode suivant l'une des revendications 1 à 3 pour le dosage de thyroxine libre, caractérisée en ce que le premier composant est un dérivé marqué de thyroxine ayant une capacité réduite de liaison vis-à-vis d'au moins l'une des protéines de liaison physiologiques, le deuxième composant est un antisérum de thyroxine et le troisième composant est un analogue d'hormones thyroïdiennes et/ou une solution de sel.

9. Méthode suivant l'une des revendications 7 ou 8, caractérisée en ce que le premier composant est marqué à l'iode $^{125}$I radio-actif.

10. Méthode suivant l'une des revendications 1 à 3 ou 8, caractérisée en ce que le premier composant est marqué avec un groupe connu doué de propriétés luminescentes.

Fig. 1 : Typische Standardkurve eines FT3-RIA

Fig. 2 : Typische Standardkurve eines FT4-RIA